# EUROPEAN PATENT SPECIFICATION

(11) **EP 0 711 543 B1**
(45) Date of publication and mention of the grant of the patent: **19.02.2003**
(21) Application number: 94308349.3
(22) Date of filing: 11.11.1994
(51) Int. Cl.: A61K 7/16, A61K 7/22

(54) **Oral preparations**
Orale Zubereitungen
Compositions orales

(43) Date of publication of application: 15.05.1996
(73) Proprietor: UNILEVER PLC, London EC4P 4BQ (GB); Unilever N.V., 3130 AC Vlaardingen (NL)
(72) Inventor: Gibbs, Christopher David, Bromborough, Wirral L63 OPH (GB)
(74) Representative: van Gent, Jan Paulus

(56) References cited:
- EP-A- 0 117 905
- WO-A-93/10776
- US-A- 4 053 638

## Description

This invention relates to oral preparations and in particular to oral preparations having an anti-caries activity.

It is well-known that various water-soluble fluorine-containing compounds are useful for combating dental caries. Examples are sodium monofluorophosphate, sodium fluoride and stannous fluoride.

There have been proposals to combat dental caries through the use in oral compositions of agents which do not contain fluorine. One of the ways to combat dental caries is to induce a pH-rise in the plaque, or to buffer the plaque-pH. For that purpose, urea has been suggested and used in oral preparations (J. Am. Dent. Assoc. 49, (1954), pages 185-190). Similarly, arginine has been proposed to buffer plaque-pH and to produce a pH-rise effect (US-A-4,154,813).

In our quest for other agents that induce a pH-rise in the plaque, we discovered that pyruvic acid or a salt thereof has a pH-rise effect when mixed with S.mutans FA-1 and Lactobacillus acidophilius and salivary sediment-systems containing mixtures of oral bacteria.

Pyruvic acid has been proposed, amongst a variety of other agents, as an anti-caries agent in JP-A-59/029618. According to this reference, the pyruvic acid has an anti-microbial activity against S.mutans in the oral cavity. A pH-rise effect is not disclosed. We have now surprisingly found, that the combination of pyruvic acid or an orally acceptable salt thereof with urea and/or arginine produces an additive pH-buffering effect.

Consequently, the present invention relates to oral preparations having an anti-caries activity, comprising pyruvic acid or an orally-acceptable salt thereof, characterised in that it further comprises urea and/or arginine.

The orally-acceptable salts of pyruvic acid are the alkalimetal salts such as the sodium and potassium salts. Other suitable salts are litium, magnesium, calcium, strontium, copper, zinc, stannous, lanthanium, yterbium, scandium, europium.

The amount of the pyruvic acid or salt thereof, used in the present invention, ranges from 1 to 50 % by weight, preferably from 2 to 25 % by weight, and most preferably 5-10 % by weight.

The arginine, used in the present invention, is normally present in an amount of 1 to 50 % by weight, preferably 2 to 25 % by weight, and particularly preferably 5-10 % by weight. The urea is normally present in an amount of 1-20 % by weight, preferably 1-10 % by weight. Derivatives of urea and arginine may also be used, e.g. sialin, small peptides with 2-4 amino acids, one of which is arginine of the type AA-arg and arg-AA, wherein AA is glycine, leucine, isoleucine, tyrosine, serine, as described in US-A-4,154,813, and compounds according to US-A-4,477,429 of the formula:

CH₃(CH₂)yNH-CH(COOH)-(CH₂)₃-NH-C-(:NH)-NH₂

where y = 6-29, such as N-alpha-octylarginine and N-alpha-decylarginine.

Together with the pyruvic acid or salt thereof and urea and/or arginine, the oral product of the invention will contain other conventional ingredients well-known to those skilled in art depending on the form of the oral product. For instance, in the case of an oral product in the form of a dentifrice cream or paste, the product will comprise an humectant-containing liquid phase and a binder or thickener which acts to maintain the particulate solid abrasive in stable suspension in the liquid phase. A surfactant and a flavouring agent are also usual ingredients of commercially acceptable dentifrices.

Humectants commonly used are glycerol and sorbitol syrup (usually comprising an approximately 70 % solution). However, other humectants are known to those in the art including propylene glycol, lactitol and hydrogenated corn syrup. The amount of humectant will generally range from about 10 to 85 % by weight of the dentifrice. The remainder of the liquid phase will consist substantially of water.

Likewise, numerous binding or thickening agents have been indicated for use in dentifrices, preferred ones being sodium carboxymethylcellulose and xanthan gum. Others include natural gum binders such as gum tragacanth, gum karaya and gum arabic, Irish moss, alginates and carrageenans. Silica thickening agents include the silica aerogels and various precipitated silicas. Mixtures of binding and thickening agents may be used. The amount of binder and thickening agent included in a dentifrice is generally between 0.1 and 10 % by weight.

It is usual to include a surfactant in a dentifrice and again the literature discloses a wide variety of suitable materials. Surfactants which have found wide use in practice are sodium lauryl sulphate, sodium dodecylbenzene sulphonate and sodium lauroylsarcosinate. Other anionic surfactants are usually present in an amount of from 0.5 to 5 % by weight of the dentifrice.

Flavours that are usually used in dentifrices are those based on oils of spearmint and peppermint. Examples of other flavouring materials used are menthol, clove, wintergreen, eucalyptus and aniseed. An amount of from 0.1 % to 5 % by weight is a suitable amount of flavour to incorporate in a dentifrice.

The oral compositions of the invention may also comprise an abrasive agent such as silica, alumina, hydrated alumina, calcium carbonate, anhydrous dicalcium phosphate, dicalcium phosphate dihydrate, water-insoluble sodium metaphosphate, calcium pyrophosphate, hydroxy apatites etc. in an amount of up to 60 % by weight.

The oral composition of the invention may include a wide variety of optional ingredients. These include an anti-plaque agent such as an anti-microbial compound for example chlorhexidine or 2,4,4'-trichloro-2'-hydroxy-diphenyl ether, or a zinc compound (see EP-A-161,898); an anti-tartar ingredient such as a condensed phosphate, e.g. an alkali metal pyrophosphate, hexametaphosphate or polyphosphate, (see US-A-4,515,772 and US-A-4,627,977) or zinc citrate (see US-A-4,100,269); sweetening agent, such as saccharin; an opacifying agent, such as titanium dioxide; a preservative, such as formalin; a colouring agent; or pH-controlling agent such as an acid, base or buffer, such as benzoic acid. Biomolecules such as enzymes, bacteriocins, anti-bodies etc. may also be included, as well as bleaching and whitening agents such as peroxy compounds.

The compositions of the present invention may also contain additional anti-caries agents, such as sodium fluoride, sodium monofluorophosphate, stannous fluoride, calcium glycerophophate, sodium trimetaphosphate, casein and casein derivatives etc..

For a fuller discussion of the formulation of oral compositions, reference is made to Harry's Cosmeticology, Seventh Edition, 1982, Edited by J.B. Wilkinson and R.J. Moore, pages 609 to 617.

The following Examples illustrate the invention. Percentages and parts are by weight.

The combination of the anti-caries agents of the invention may also be included in foodstuffs for caries reduction/prevention.

### Example 1

Salivary sediment experiments were carried out similar to those reported by Kleinberg in Arch. Oral Biol., Vol. 28, 11, (1983), page 1007.

Two panellists fasted and refrained from oral hygiene for 12 hours before the experiment. Fifteen millilitres of stimulated saliva were then collected and placed in weighed centrifuge tubes. The sediment was centrifuged and washed twice with KC1 solution. It was diluted with the same weight of KC1 solution and incubated at 37°C for three hours.

All the solutions were taken to pH 7 and mixed together. The sediment was then also set to pH 7 and this was then added to the mixture of solutions. The pH of the solutions were then measured at 0, 15, 30, 45, 60 90, 120, 150, 180 and 210 minutes. The data was then plotted on a pH v time graph.

| | | |
|---|---|---|
| Final concentrations of solutions | KC1 | 135mM |
| | Glucose | 2.8mM |
| | Pyruvate | 33mM |
| | Arginine | 33mM |
| | Urea | 1.5mM |
| Amounts of solutions added | Glucose | 20µl |
| | Test | 100µl |
| | Sediment | 500µl |
| | KC1 | 2.48ml, 2.38ml or 2.28ml |
| | (Dependant on the amount of test solution added) | |

For the solutions containing pyruvate and another agent 100µl of each solution were added so the final concentrations were the same.

If required to ensure a large pH drop extra glucose was added after 30 minutes.

The following results were obtained:

| Time (mins) | Water | Pyruvate | Arginine | Urea | Pyr + Arg | Pyr + Urea |
|---|---|---|---|---|---|---|
| 0 | 7.32 | 7.32 | 7.40 | 7.33 | 7.44 | 7.36 |
| 15 | 6.75 | 6.67 | 6.88 | 7.12 | 6.77 | 6.99 |
| 30 | 6.61 | 6.67 | 6.79 | 6.95 | 6.72 | 6.99 |
| 45 | 6.42 | 6.58 | 6.71 | 6.91 | 6.68 | 6.97 |
| 60 | 6.40 | 6.67 | 6.65 | 6.88 | 6.73 | 6.91 |
| 90 | 5.72 | 6.39 | 6.39 | 6.56 | 6.59 | 6.78 |
| 120 | 5.36 | 6.30 | 6.28 | 6.40 | 6.63 | 6.70 |
| 150 | 5.25 | 6.23 | 6.30 | 6.50 | 6.57 | 6.72 |
| 180 | 5.33 | 6.22 | 6.59 | 6.58 | 6.62 | 6.68 |
| 210 | 5.35 | 6.14 | 6.83 | 6.71 | 6.73 | 6.71 |

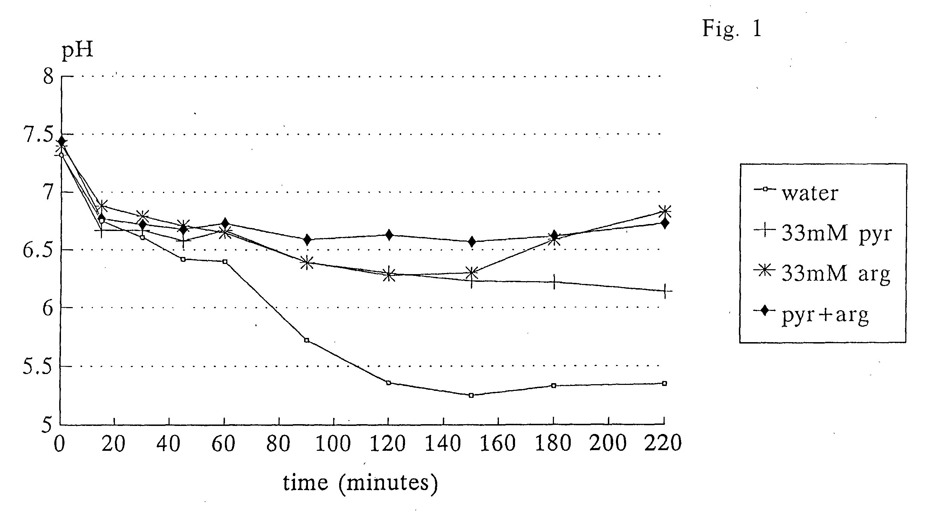

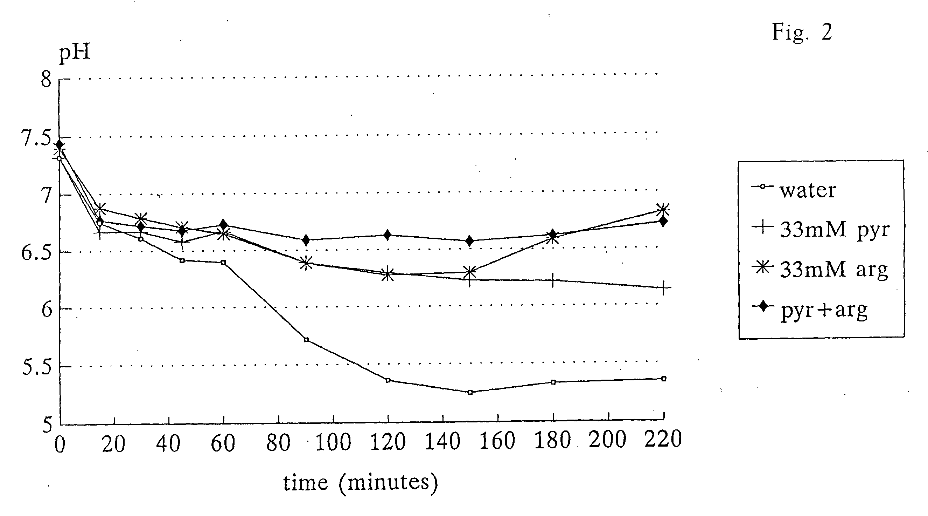

Fig. 1 and 2 represent these data.

## Claims

1. An oral preparation having an anti-caries activity, comprising pyruvic acid or an orally-acceptable salt thereof **characterised in that** it further comprises urea or a derivative thereof and/or arginine or a derivative thereof.

2. A preparation according to claim 1, comprising 1-50 % by weight of pyruvic acid or an orally acceptable salt thereof, and 1-20 % by weight of urea or a derivative thereof and/or 1-50 % by weight of arginine or a derivative thereof.

3. A preparation according to claim 2, wherein the weight percentages are 5-10 %, 5-10 % and 1-10 % respectively.

4. Use of a mixture of pyruvic acid or an orally acceptable salt thereof and urea and/or arginine or a derivative of urea and/or arginine for the preparation of an anti-caries agent.

## Patentansprüche

1. Orale Zubereitung mit einer Antikarieswirkung, umfassend Brenztraubensäure oder ein oral verträgliches Salz davon, **dadurch gekennzeichnet, dass** sie weiterhin Harnstoff oder ein Derivat davon und/oder Arginin oder ein Derivat davon umfasst.

2. Zubereitung nach Anspruch 1, umfassend 1-50 Gewichtsprozent Brenztraubensäure oder ein oral verträgliches Salz davon und 1-20 Gewichtsprozent Harnstoff oder ein Derivat davon und/oder 1-50 Gewichtsprozent Arginin oder ein Derivat davon.

3. Zubereitung nach Anspruch 2, wobei die Gewichtsprozentsätze 5-10%, 5-10% bzw. 1-10% sind.

4. Verwendung eines Gemisches von Brenztraubensäure oder einem oral verträglichen Salz davon und Harnstoff und/oder Arginin oder einem Derivat von Harnstoff und/oder Arginin zur Herstellung eines Antikariesmittels.

## Revendications

1. Préparation orale ayant une activité anti-caries, comprenant de l'acide pyruvique ou un de ses sels oralement acceptables **caractérisée en ce qu'**elle comprend en outre de l'urée ou un de ses dérivés et/ou de l'arginine ou un de ses dérivés.

2. Préparation selon la revendication 1, comprenant 1 à 50% en poids d'acide pyruvique ou d'un de ses sels oralement acceptables, et 1 à 20% en poids d'urée ou d'un de ses dérivés et/ou 1 à 50% en poids d'arginine ou d'un de ses dérivés.

3. Préparation selon la revendication 2, dans laquelle les pourcentages en poids sont respectivement 5 à 10%, 5 à 10% et 1 à 10%.

4. Utilisation d'un mélange d'acide pyruvique ou d'un de ses sels oralement acceptables et d'urée et/ou d'arginine ou d'un dérivé d'urée et/ou d'arginine pour la préparation d'un agent anti-caries.
